# EUROPÄISCHE PATENTSCHRIFT

(11) **EP 2 258 419 B1**
(45) Veröffentlichungstag und Bekanntmachung des Hinweises auf die Patenterteilung: **08.01.2014**
(21) Anmeldenummer: 10010373.8
(22) Anmeldetag: 25.02.1999
(51) Int. Cl.: A61M 1/16, A61M 39/10

(54) **Dialysegerät mit Vorrichtung zur Herstellung von Dialyselösungen**
Dialysis system with apparatus for preparing dialysates
Système de dialyse avec appareil d'élaboration de dialysates

(30) Priorität: 01.04.1998 DE 19814687
(43) Veröffentlichungstag der Anmeldung: 08.12.2010
(62) Teilanmeldung aus: 04012755.7
(73) Patentinhaber: Fresenius Medical Care Deutschland GmbH, 61352 Bad Homburg (DE)
(72) Erfinder: Dönig, Rainer, 60489 Frankfurt/M (DE); Döpper, Joachim, 35428 Langgöns (DE); Schulz, Wolfgang, 66606 St. Wendel (DE)
(74) Vertreter: Herrmann, Uwe

(56) Entgegenhaltungen:
- EP-A- 0 311 848
- EP-A- 0 575 970
- WO-A-96/25214
- US-A- 5 807 316

## Beschreibung

Die vorliegende Erfindung betrifft ein Dialysegerät mit einer Vorrichtung zur Herstellung von Dialyselösungen. Die Erfindung betrifft ferner ein Verfahren zum Erkennen des Anschlusses eines Lösungsbestandteils-Vorratsbehälters an ein medizinisches Gerät.

Ein gattungsgemäßes Dialysegerät ist beispielsweise aus der WO 92/11046 bekannt. Hier wird ein Dialysegerät mit einer Vorrichtung offenbart, mit der Dialyselösungen gewünschter Beschaffenheit hergestellt und in den Dialyseflüssigkeitskreislauf des Gerätes eingespeist werden. Zur Herstellung der benötigten Lösungen werden Tabletten verwendet, die die erforderlichen Bestandteile der Dialyselösungen enthalten und die bei Bedarf in eine wasserenthaltende Mischkammer eingegeben und darin in Wasser gelöst werden. Die Tabletten können beispielsweise in einem über der Mischvorrichtung angeordneten Magazin vorgesehen sein. Ein den Bedürfnissen der Patienten angepaßter Konzentrationsverlauf der chemischen Bestandteile der Dialyselösungen wird dadurch erreicht, daß zu vorgebbaren Zeitpunkten unterschiedliche Wirkstoffmengen oder -arten enthaltende Tabletten dem Lösevorgang und anschließend dem Dialyseflüssigkeitskreislauf zugeführt werden.

Um Fehler bei der Art und Konzentration der verabreichten Dialyselösungen zu vermeiden und um dem Prozeß überwachen zu können, weisen die Tabletten einen Strichcode auf, der mittels einer Lesevorrichtung des Dialysegerätes erfaßt werden kann. Ein Nachteil dieser Vorrichtung besteht darin, daß entsprechend der Breite des anzuwendenden Konzentrationsbereichs eine Vielzahl unterschiedliche Wirkstoffkonzentrationen enthaltende Tabletten hergestellt und mit einem Strichcode versehen werden müssen, was deren Herstellung entsprechend aufwendig und teuer gestaltet. Zudem sind aufgrund der festen Beschaffenheit der Tabletten Vorrichtungen zur Zugabe der Tabletten in ein geeignetes Lösemittel sowie zum Lösen der Tabletten notwendig, wodurch die Dialysegeräte einen verhältnismäßig komplexen Aufbau annehmen. WO96/25214 offenbart ein Dialysegerät und ein Verfahren gemäß dem Oberbegriff der Ansprüche 1 und 9.

Es ist die Aufgabe der vorliegende Erfindung ein Dialysegerät zur Verfügung zu stellen, mit dem auf einfache Weise unterschiedlich konzentrierte Dialyseflüssigkeiten zuverlässig herstellbar sind.

Diese Aufgabe wird ausgehend von einem gattungsgemäßen Dialysegerät durch die Merkmale des Anspruchs 1 gelöst. Dadurch wird es möglich, einfach und zuverlässig unterschiedlich konzentrierte Dialyselösungen herzustellen, wobei als Ausgangslösungen übliche Standardlösungen eingesetzt werden können. Entsprechend entfällt die Notwendigkeit, zahlreiche unterschiedlich konzentrierte Standardlösungen bereitzustellen, um eine den Bedürfnissen des Patienten optimal angepaßte Dialyse durchführen zu können.

Die Konnektoren oder den Konnektoren benachbarte Bereiche eines Verbindungsschlauches weisen Kennzeichnungsmittel auf, die mittels der Erfassungsvorrichtung detektierbar sind. Dadurch werden Fehler bei der Zuordnung der Vorratsbehälter zu den Anschlüssen der Vorrichtung sicher vermieden. Aufgrund der die Vorratsbehälter identifizierenden Kennzeichnungsmittel erfaßt das Dialysegerät automatisch die Art und/oder Menge der enthaltenen Lösung, so daß unabhängig von der Wahl des Anschlusses stets eine eindeutige Identifizierung erfolgt.

Der Bediener des Dialysegerätes steckt in beliebiger Reihenfolge die Konnektoren der benötigten Vorratsbehälter auf die Anschlüsse der Vorrichtung und gibt beispielsweise über eine Steuereinrichtung die gewünschten Konzentrationen bzw. Konzentrationsgradienten der herzustellenden Dialyseflüssigkeit ein. Das erfindungsgemäße Dialysegerät erkennt anhand der Kennzeichnungsmittel selbständig welcher Anschluß mit welchen Lösungen beaufschlagt wird und führt entsprechend beispielsweise durch die Schaltung von Pumpen oder Ventilen den gewünschten Herstell- bzw. Mischvorgang durch. Eine möglicherweise zur Gefährdung des Patienten führende Verwechslung von Anschlüssen und somit die Herstellung von Dialyselösungen mit unerwünschten Wirkstoffen oder Wirkstoffmengen kann somit sicher vermieden werden. Ein weiterer Vorteil der erfindungsgemäßen Vorrichtung ergibt sich daraus, daß auch zur Herstellung zahlreicher unterschiedlich konzentrierter Dialyseflüssigkeiten stets nur eine geringe Anzahl von Standardlösungen notwendig ist, was produktionstechnisch und logistisch zu erheblichen Vereinfachungen führt.

Gemäß einer bevorzugten Ausgestaltung der vorliegenden Erfindung sind als Vorratsbehälter Lösungsbeutel vorgesehen, wobei die Lösungsbeutel einen Verbindungsschlauch umfassen, an dessen Ende ein Konnektor vorgesehen ist. Die Lösungsbeutel enthalten beispielsweise Standardlösungen, die nur in geringer Anzahl zur Verfügung gestellt werden müssen.

Das Kennzeichnungsmittel kann an einer vorgebbaren Position des Konnektors vorgesehen sein und die Erfassungsvorrichtung kann derart ausgeführt und/oder angeordnet sein, daß neben der Art auch die Position des Kennzeichnungsmittels erfaßbar ist. Die Schaffung des Kennzeichnungsmittels auf dem Konnektor hat den Vorteil, daß unabhängig von der Länge oder Anzahl der Verbindungsschläuche eine Verwechslung der Vorratsbehälter bzw. eine fehlerhafte Zuordnung zu den Anschlüssen ausgeschlossen ist. Vielmehr ist es unnötig, daß der Bediener darauf achtet, welcher Konnektor mit welchem Anschluß verbunden wird, da das erfindungsgemäße Dialysegerät eine automatische Erkennung des Konnektors und des damit in Verbindung stehenden Vorratsbehälters bzw. Lösungsbeutels durchführt. Erfindungsgemäß ist es darüber hinaus vorgesehen, daß die Erfassungsvorrichtung die Position des Konnektors ermittelt, wodurch es dem Bediener möglich wird, zu erkennen, daß ein Konnektor nicht vollständig auf den Anschluß aufgesteckt ist. In diesem Fall wäre das Kennzeichnungsmittel gegenüber der vollständig aufgesteckten Position geringfügig verschoben, was durch die Erfassungsvorrichtung detektiert wird.

Erfindungsgemäß sind Mittel vorgesehen, mittels derer ein fehlerhaftes Aufstecken eines Konnektors auf einen Anschluss der Vorrichtung erkennbar ist.

Weiterhin sind Mittel vorgesehen, mittels derer eine während des Betriebes erfolgende Diskonnektion eines Konnektors von einem Anschluss der Vorrichtung erkennbar ist.

Die vorgenannten Mittel werden durch die erfindungsgemäße Erfassungsvorrichtung gebildet.

Erfindungsgemäß umfasst das Kennzeichnungsmittel einen Strichcode. Derartige Codierungen sind in einer großen Vielzahl herstellbar und können problemlos auf die Konnektoren oder die Verbindungsschläuche aufgebracht werden. Darüber hinaus ermöglicht die Verwendung eines Strichcodes die Detektion nicht nur der Art der angeschlossenen Lösung, sondern auch die Erfassung einer Diskonnektion bzw. das Erkennen eines fehlerhaft aufgesteckten Konnektors.

In weiterer Ausgestaltung der vorliegenden Erfindung ist vorgesehen, daß der Strichcode derart auf den Konnektoren angeordnet ist, daß die Streifen des Strichcodes in Umfangsrichtung des Konnektors verlaufen. Dadurch wird sichergestellt, daß ein Verdrehen des Konnektors nicht zu einer fehlenden oder mangelhaften Erfassung durch die Erfassungsvorrichtung führen kann, da sich der Strichcode über den gesamten Umfang erstreckt. Darüber hinaus ergibt sich der Vorteil, daß sich der Strichcode im Gegensatz zu in Längsrichtung des Konnektors verlaufenden Markierungen von der Erfassungsvorrichtung vollständig erfassen läßt.

Gemäß einer bevorzugten Ausgestaltung der vorliegenden Erfindung umfaßt das Kennzeichnungsmittel Informationen über Art und Volumen der zu dosierenden Lösungsbestandteile des Vorratsbehälters. Dadurch wird sichergestellt, daß nicht nur die Wirkstoffbeschaffenheit kontrollierbar ist, sondern auch der Zeitpunkt bestimmbar, ab dem möglicherweise ein Wechsel der Vorratsbehälter durchzuführen ist. Insbesondere ist es möglich, daß in diesem Fall die Erfassungsvorrichtung bereits zu Beginn der Behandlung ein für die anstehende Dialyse zu geringes Volumen der zu verabreichenden Lösung der Vorratsbehälter feststellt, was entsprechend dem Bediener angezeigt werden kann. Damit kann vermieden werden, daß eine Unterbrechung des Dialysevorganges dadurch notwendig wird, daß die Vorratsbehälter gewechselt werden müssen.

Gemäß einer bevorzugten Ausgestaltung der vorliegenden Erfindung ist eine Auswerteeinheit vorgesehen, die mit der Erfassungsvorrichtung verbindbar ist, wobei die Auswerteeinheit derart ausgeführt ist, daß Sollwerte für die Menge und/oder Beschaffenheit der in den Vorratsbehältern aufnehmbaren Lösungsbestandteilen der Dialyselösung speicherbar und mit den von der Erfassungsvorrichtung ermittelten Istwerten vergleichbar sind. Der Bediener des Dialysegerätes gibt vor Beginn der Behandlung die erforderlichen Daten beispielsweise für die gewünschten Wirkstoffkonzentrationen, -mengen und -gradienten ein, wonach diese Werte in der Auswerteeinheit als Sollwerte abgelegt werden. Anschließend werden die Vorratsbehälter mit den Anschlüssen der Vorrichtung mittels der Konnektoren verbunden und die Erfassungsvorrichtung ermittelt mit Hilfe der Kennzeichnungsmittel die Art und beispielsweise das Volumen der angeschlossenen Lösungen. Im Anschluß daran kann entsprechend der Vorgaben des Bedieners eine gewünschte Mischung bzw. ein zeitliches Profil von Wirkstoffkonzentrationen hergestellt werden, wenn die Auswerteeinheit keine Abweichungen zwischen den Vorgaben des Bedieners und den tatsächlich verwendeten Lösungen erkennt.

Besonders vorteilhaft ist es, wenn Signal- oder Absperrmittel vorgesehen sind, die mit der Auswerteeinheit verbindbar sind. Beispielsweise ist es möglich, daß beim unvollständigen Aufstecken eines Konnektors oder bei der Verwendung falscher Lösungsbestandteile ein optisches oder akustisches Signal ausgegeben wird oder daß Absperrmittel, wie z.B. Ventile, geschaltet werden, wodurch die Verabreichung der fehlerhaft angeschlossenen oder falschen Lösungen verhindert wird.

Die Absperrmittel können Mittel zur mechanischen und/oder elektrischen Absperrung von Leitungen umfassen. Erkennt die Auswerteeinheit eine Abweichung zwischen Soll- und Istwerten hinsichtlich der Art oder Menge der Wirkstoffe, werden die Absperrmittel derart betätigt, daß beispielsweise entweder eine Zufuhrleitung zum Dialysegerät abgesperrt wird, wodurch eine Abgabe von Dialyselösung in den Dialysator verhindert wird. Ebenso ist es möglich, daß die elektrische Versorgung beispielsweise der Pumpe des Dialysekreislaufes nicht aktivierbar ist, solange die Auswerteeinheit eine fehlende Übereinstimmung von Soll- und Istwerten feststellt.

Beschrieben wird ferner ein Konnektor zum Anschluß eines Vorratsbehälters mit Lösungsbestandteilen an ein medizinisches Gerät. Zur Identifikation des Konnektors weist dieser Kennzeichnungsmittel auf. Die Kennzeichnungsmittel umfassen einen Strichcode.

Besonders vorteilhaft ist es, wenn der Strichcode umlaufend ausgeführt ist. Daraus ergibt sich der Vorteil, daß beim Aufstecken des Konnektors nicht auf eine bestimmte Drehposition geachtet werden muß, da sich der Strichcode erfindungsgemäß umlaufend um den gesamten Umfang des Konnektors erstreckt. Ein weiterer Vorteil einer derartigen Ausführung besteht darin, daß der umlaufende Strichcode im Gegensatz zu in Längsrichtung des Konnektors verlaufenden Markierungen von einer Erfassungsvorrichtung stets vollständig und zuverlässig erfaßt werden kann.

Die vorliegende Erfindung betrifft ferner ein Verfahren zum Erkennen eines Anschlusses eines Lösungsbestandteils-Vorratsbehälters gemäß Anspruch 9. Daraus ergibt sich der Vorteil, daß eine Verwechslung der erfindungsgemäß gekennzeichneten Konnektoren bzw. der Anschlüsse nicht mehr möglich ist. Darüber hinaus ist eine mangelhafte Verbindung des Konnektors mit einem Gegenstück bzw. eine während des Dialysevorgangs auftretende Diskonnektion zuverlässig und schnell erkennbar.

Weitere Vorteile und Einzelheiten der vorliegenden Erfindung werden anhand eines in der Zeichnung dargestellten Ausführungsbeispiels näher erläutert. Es zeigt:

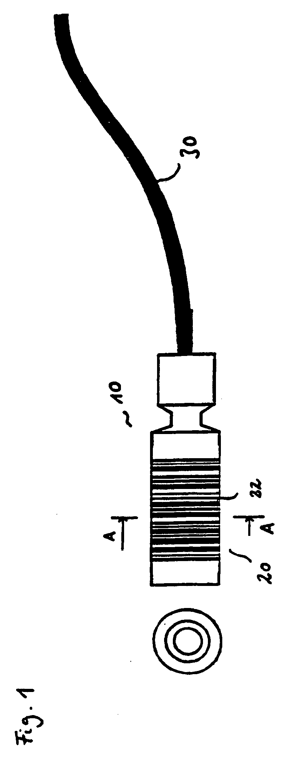

- Fig. 1:: Ein an einem Verbindungsschlauch zum Lösungsbeutel angeordneten Konnektor mit Strichcode.

Fig. 1 zeigt den Konnektor 10 in einer Schnittdarstellung (links) gemäß der Linie A-A der in Fig. 1 rechts dargestellten Seitenansicht. Der Konnektor 10 ist am Ende des Verbindungsschlauches 30 angeordnet, wobei der Verbindungsschlauch 30 an seinem anderen (nicht dargestellten) Ende mit einem die erforderlichen Lösungsbestandteile enthaltenden Lösungsbeutel verbunden ist.

Der Konnektor 10 weist als Kennzeichnungsmittel 20 den Strichcode 22 auf, wobei die Streifen des Strichcodes 22 in Umfangsrichtung des Konnektors 10 verlaufen. Dadurch wird sichergestellt, daß unabhängig davon, ob der Konnektor 10 beim Aufstecken auf einen Anschluß verdreht ist, eine Detektion des Strichcodes 22 durch die Erfassungsvorrichtung möglich ist.

Der Strichcode 22 enthält beispielsweise Informationen über die Art und Menge der Lösung, die in dem damit verbundenen Lösungsbeutel aufgenommen ist. Der Bediener des Dialysegerätes kann nach der Auswahl der geeigneten Lösungen die Konnektoren auf beliebige Anschlüsse der Dialysevorrichtung aufstecken, da erfindungsgemäß eine Erfassung der Art des Kennzeichnungsmittels 20 bzw. ein Lesen des Strichcodes 22 erfolgt. Aufgrund dieser Identifikation der Lösungsbeutel ist eine Verwechslung von Lösungen, die zur Abgabe fehlerhaft hergestellter Dialyseflüssigkeiten führen könnte, ausgeschlossen. Die Anordnung des Strichcodes 22 auf dem Konnektor 10 gemäß Fig. 1 ermöglicht es ferner, zu erkennen, ob der Konnektor 10 ordnungsgemäß und vollständig auf dem Anschluß der Vorrichtung aufgesteckt ist. Ist dies nicht der Fall, ermittelt die Erfassungsvorrichtung ein Streifenmuster, das dem Streifenmuster des vollständig aufgesteckten Konnektors 10 aufgrund der Verschiebung nicht entspricht. Dadurch wird das fehlerhafte Aufstecken sowie eine während des Betriebes erfolgende Diskonnektion sicher und zuverlässig erkannt.

Das erfindungsgemäße Dialysegerät ermöglicht es, eine erhöhte Behandlungsqualität dadurch zu erreichen, daß die verabreichten Dialyselösungen, insbesondere Peritonealdialyselösungen, individuell herstellbar und entsprechend für den Patienten optimal abstimmbar sind. Insbesondere können Standardlösungen eingesetzt werden, die in nur verhältnismäßig geringer Anzahl zur Verfügung gestellt werden müssen. Ein erhöhter Bedienungskomfort und eine höhere Betriebssicherheit wird dadurch erreicht wird, daß die Lösungsbeutel bzw. Vorratsbehälter in beliebiger Reihenfolge auf die Anschlüsse aufgesteckt werden können, da die eindeutige Identifikation der Lösungsbeutel bzw. deren Inhalt durch das erfindungsgemäße Gerät selbständig vorgenommen wird.

## Patentansprüche

1. Dialysegerät mit einer Vorrichtung zur Herstellung von Dialyselösungen, wobei die Vorrichtung eine Erfassungsvorrichtung, wenigstens zwei Anschlüsse sowie wenigstens zwei austauschbare Vorratsbehälter zur Aufnahme der zu dosierenden Lösungsbestandteile umfaßt, die jeweils mit mindestens einem Konnektor (10) verbunden sind, wobei die Konnektoren (10) mit den Anschlüssen verbindbar sind, und wobei die Konnektoren (10) Kennzeichnungsmittel (20) aufweisen, die mittels der Erfassungsvorrichtung detektierbar sind, und wobei das Kennzeichnungsmittel (20) einen Strichcode umfaßt,
**dadurch gekennzeichnet, dass**
mittels der Erfassungsvorrichtung ein fehlerhaftes Aufstecken eines Konnektors (10) auf einen Anschluss der Vorrichtung und/oder eine während des Betriebes erfolgende Diskonnektion eines Konnektors (10) von einem Anschluss der Vorrichtung erkennbar ist.

2. Dialysegerät nach Anspruch 1, **dadurch gekennzeichnet, daß** als Vorratsbehälter Lösungsbeutel vorgesehen sind und daß die Lösungsbeutel einen Verbindungsschlauch (30) umfassen, an dessen Ende ein Konnektor (10) vorgesehen ist.

3. Dialysegerät nach Anspruch 1 oder 2, **dadurch gekennzeichnet, daß** das Kennzeichnungsmittel (20) an einer vorgebbaren Position des Konnektors (10) vorgesehen ist und die Erfassungsvorrichtung derart ausgeführt und/oder angeordnet ist, daß neben der Art auch die Position des Kennzeichnungsmittels (20) erfaßbar ist.

4. Dialysegerät nach Anspruch 1, **dadurch gekennzeichnet, daß** der Strichcode (22) derart auf den Konnektoren (10) angeordnet ist, daß die Streifen des Strichcodes (22) in Umfangsrichtung des Konnektors (10) verlaufen.

5. Dialysegerät nach einem oder mehreren der Ansprüche 1 bis 4, **dadurch gekennzeichnet, daß** das Kennzeichnungsmittel (20) Informationen über Art und Volumen der zu dosierenden Lösungsbestandteile des Vorratsbehälters umfaßt

6. Dialysegerät nach einem oder mehreren der Ansprüche 1 bis 5, **dadurch gekennzeichnet, daß** eine Auswerteeinheit vorgesehen ist, die mit der Erfassungsvorrichtung verbindbar ist, wobei die Auswerteeinheit derart ausgeführt ist, daß Sollwerte für die Menge und/oder Beschaffenheit der in den Vorratsbehältern aufnehmbaren Lösungsbestandteilen der Dialyselösung speicherbar und mit den von der Erfassungsvorrichtung ermittelten Istwerten vergleichbar sind.

7. Dialysegerät nach Anspruch 6, **dadurch gekennzeichnet, daß** Signal- oder Absperrmittel vorgesehen sind, die mit der Auswerteeinheit verbindbar sind.

8. Dialysegerät nach Anspruch 7, **dadurch gekennzeichnet, daß** die Absperrmittel Mittel zu mechanischen und/oder elektrischen Absperrung von Leitungen umfassen.

9. Verfahren zum Erkennen eines Anschlusses eines Lösungsbestandteils-Vorratsbehälters an ein medizinisches Gerät, wobei ein Konnektor (10), der mit einem Anschluss des medizinischen Gerätes verbindbar ist, vorgesehen ist, wobei der Konnektor (10) mit Kennzeichnungsmitteln (20) versehen wird und auf ein Gegenstück aufgesteckt wird und eine Erfassungsvorrichtung die Art und Position des Konnektors (10) bestimmt, wobei die Kennzeichnungsmittel einen Strichcode umfassen,
**dadurch gekennzeichnet dass**
mittels der Erfassungsvorrichtung ein fehlerhaftes Aufstecken des Konnektors (10) auf einen Anschluss des medizinischen Gerätes und/oder eine während des Betriebes erfolgende Diskonnektion des Konnektors von einem Anschluss des medizinischen Gerätes erkennbar ist.

## Claims

1. A dialyser having an apparatus for preparing dialysis solutions, wherein the apparatus comprises a detection apparatus, at least two ports as well as at least two replaceable storage containers for receiving the solution components to be metered which are each connected to at least one connector (10), wherein the connectors (10) can be connected to the ports and wherein the connectors (10) have marking means (20) which can be detected by means of the detection apparatus and wherein the marking means (20) comprises a bar code (22),
**characterised in that**
an incorrect plugging of a connector (10) onto a port of the apparatus and/or a disconnection of a connector (10) from a port of the apparatus taking place during operation can be recognised by means of the detection apparatus.

2. A dialyser in accordance with claim 1, **characterised in that** solution bags are provided as the storage containers; and **in that** the storage containers comprise a connection hose (30) at whose end a connector (10) is provided.

3. A dialyser in accordance with claim 1 or 2, **characterised in that** the marking means (20) is provided at a predefinable position of the connector (10) and the detection apparatus is designed and/or arranged such that the position of the marking means (20) can also be detected in addition to the type thereof.

4. A dialyser in accordance with claim 1, **characterised in that** the bar code (22) is arranged on the connectors (10) such that the lines of the bar code (22) extend in the peripheral direction of the connector (10).

5. A dialyser in accordance with one or more of the claims 1 to 4, **characterised in that** the marking means (20) comprises information on the type and volume of the solution components of the storage container to be metered.

6. A dialyser in accordance with one or more of the claims 1 to 5, **characterised in that** an evaluation unit is provided which can be connected to the detection apparatus, with the evaluation unit being designed such that desired values for the quantity and/or property of the solution components of the dialysis solution which can be received in the storage containers can be stored and can be compared with the actual values determined by the detection apparatus.

7. A dialyser in accordance with claim 6, **characterised in that** signal means or blocking means are provided which can be connected to the evaluation unit.

8. A dialyser in accordance with claim 7, **characterised in that** the blocking means comprise means for the mechanical and/or electrical blocking of lines.

9. A method of recognising a port of a solution component storage container at a medical device, wherein a connector (10) is provided which can be connected to a port of the medical device, wherein the connector (10) is provided with marking means (20) and is plugged onto a counter piece, and a detection apparatus determines the type and position of the connector (10), wherein the marking means (20) comprise a bar code,
**characterised in that**
an incorrect plugging of the connector (10) onto a port of the medical device and/or a disconnection of a connector from a port of the medical device taking place during operation can be recognised by means of the detection apparatus.

## Revendications

1. Appareil de dialyse comprenant un dispositif de production de dialysats, dans lequel le dispositif comprend un dispositif de saisie, au moins deux raccordements et au moins deux récipients de stockage remplaçables destinés à recevoir les composants des dialysats à doser, qui sont chacun reliés avec au moins un connecteur (10), étant entendu que les connecteurs (10) peuvent être reliés avec les raccordements, étant entendu que les connecteurs (10) présentent des moyens d'identification (20) qui peuvent être détectés au moyen du dispositif de saisie et étant entendu que le moyen d'identification (20) comprend un code à barres,
**caractérisé**
**en ce qu'**un montage défectueux d'un connecteur (10) sur un raccordement du dispositif et/ou une déconnexion d'un connecteur (10) d'un raccordement du dispositif survenant pendant le fonctionnement peuvent être déterminés au moyen du dispositif de saisie.

2. Appareil de dialyse selon la revendication 1, **caractérisé en ce que** des sachets de solution sont prévus comme récipients de stockage et **en ce que** les sachets de solution comprennent un tuyau de raccordement (30) à l'extrémité duquel est prévu un connecteur (10).

3. Appareil de dialyse selon la revendication 1 ou 2, **caractérisé en ce que** le moyen d'identification (20) est prévu à une position du connecteur (10) pouvant être prédéfinie et le dispositif de saisie est réalisé et/ou agencé de telle sorte qu'en plus du type, la position du moyen d'identification (20) peut également être saisie.

4. Appareil de dialyse selon la revendication 1, **caractérisé en ce que** le code à barres (22) est agencé sur les connecteurs (10) de telle sorte que les barres du code à barres (22) s'étendent dans la direction périphérique du connecteur (10).

5. Appareil de dialyse selon l'une ou plusieurs des revendications 1 à 4, **caractérisé en ce que** le moyen d'identification (20) comprend des informations sur le type et le volume des composants de la solution du récipient de stockage devant être dosés.

6. Appareil de dialyse selon l'une ou plusieurs des revendications 1 à 5, **caractérisé en ce qu'**il est prévu une unité d'analyse qui peut être connectée au dispositif de saisie, étant entendu que l'unité d'analyse est réalisée de telle sorte que des valeurs de consigne pour la quantité et/ou l'état des composants du dialysat pouvant être contenus dans les récipients de stockage peuvent être enregistrées et comparées avec les valeurs effectives calculées par le dispositif de saisie.

7. Appareil de dialyse selon la revendication 6, **caractérisé en ce qu'**il est prévu des moyens de signal ou de blocage qui peuvent être connectés à l'unité d'analyse.

8. Appareil de dialyse selon la revendication 7, **caractérisé en ce que** les moyens de blocage comprennent des moyens permettant un blocage mécanique et/ou électrique de conduites.

9. Procédé d'identification d'un raccordement d'un récipient de stockage de composants d'une solution à un appareil médical, dans lequel il est prévu un connecteur (10) qui peut être relié à un raccordement de l'appareil médical, étant entendu que le connecteur (10) est muni de moyens d'identification (20) et monté sur une pièce opposée et qu'un dispositif de saisie détermine le type et la position du connecteur (10), les moyens d'identification comprenant un code à barres,
**caractérisé**
**en ce qu'**un montage défectueux du connecteur (10) sur un raccordement de l'appareil médical et/ou une déconnexion du connecteur d'un raccordement de l'appareil médical survenant pendant le fonctionnement peuvent être déterminés au moyen du dispositif de saisie.
